# EUROPEAN PATENT APPLICATION

(11) **EP 0 622 394 A1**
(43) Date of publication of application: **02.11.1994**
(21) Application number: 93870076.2
(22) Date of filing: 30.04.1993
(51) Int. Cl.: C08G 65/32, A61K 47/48, C07K 3/08

(54) **Reversible modification of sulfur-containing molecules with polyalkylene glycol derivatives and their use**

(71) Applicant: S.A. LABORATOIRES S.M.B., B-1080 Bruxelles (BE)
(72) Inventor: Looze, Yvan, U.L.B., Faculty of Medecine, B-1050 Bruxelles (BE); Vincentelli, Jean, U.L.B., Faculty of Medecine, B-1050 Bruxelles (BE); Brygier, Jeanne, U.L.B., Faculty of Medecine, B-1050 Bruxelles (BE); Paul, Claudine, U.L.B., Faculty of Medecine, B-1050 Bruxelles (BE); Nijs, Michelle, U.L.B., Faculty of Medecine, B-1050 Bruxelles (BE); Guermant, Claude, U.L.B., Faculty of Medecine, B-1050 Bruxelles (BE); Volant-Baeyens, Danièle, B-1050 Bruxelles (BE); Deboeck, Arthur, Puerto Rico 00778 (US); Baudier, Philippe, B-1410 Waterloo (BE)
(74) Representative: De Palmenaer, Roger

(57) **Abstract**

Poly(alkylene glycol) derivatives of the formula :

PAG [ O - V - Hg^{⊖}x^{⊕} ]ₙ

or

PAG [ O - V₁ - S - R₁ ]ₙ

wherein
- PAG: is a poly(alkylene glycol),
- x: is a counter ion,
- V, V₁: is a non polymeric organic group,
- n: is 1 or 2, and
- R₁: is selected from the group of -SO₃^{⊕}; -S-SO₃^{⊕}; -SO₂CH₃; thiopyridyl; nitrosulfenyl; otherwise substituted sulfenyl; -SO-(CH₂)ₘ-CH₃ with m = 0, 1, ...; -SOₘ-CH₂-CH=CH₂ with m = 1, 2; -SO₂-R where R is alkyl or aryl.

## Description

### Description of the prior state of the art

The chemical attachment of polyalkylene glycol (PAG) to protein is a method of choice which confers to the so modified proteins new and interesting properties. As a general rule, PAG modified proteins exhibit increased stability, increased resistance to proteolytic inactivation, increased plasmatic half-live, decreased to non existent antigenicity and immunogenicity and lower toxicity.

As a consequence of the potential usefulness of all these conferred properties, the covalent attachment of PAG chains to a variety of proteins of therapeutical interest has been attempted. Some of these protein derivatives are under clinical evaluation since they retain, at least partly, their biological activity. In March 1990, ENZON INC. even received an approval to market for a PEG - modified form of adenosine deaminase (ADAGEN™) useful in the treatment of severe combined immunodeficiency disease associated with adenosine deaminase deficiency.

The therapeutical value of proteins is known for a long time. Historically, before the development of large - scale manufacturing processes for human recombinant proteins, most proteins used for therapy purposes were derived from non - human proteins and often induced an immune response that resulted in hypersensitivity or even death.

As a consequence of this induced immune response, the protein plasmatic clearance increases and thus needing the use of higher doses so as to maintain therapeutic efficacy. It often resulted in a vicious circle of high doses inducing immune response and increased clearance, lack of efficacy which required higher doses.

An increasingly growing number of recombinant human proteins which should not have the property of inducing immune response are now commercially available. A number of these are already in use. During the evaluation of these proteins, it became obvious that the hope recombinant technology would solve all the problems associated with the therapeutical use of proteins was not yet obtained.

Indeed, some recombinant proteins such as Interleukin-2, contrary to the "natural" human protein, lack the glycosyl moiety(ies). Because of this missing part, the recombinant Interleukin-2 is poorly soluble, unstable and rapidly cleared from the plasma. In other cases, it was showed that glycosyl moiety removal demasked an epitope normally not visible by the immune system rendering recombinant human-proteins potentially immunogenic.

Finally, it should be mentioned that some potentially useful enzymes (e.g. uricase which reduces the level of uric acid in the plasma and in the urine) form human therapy are not produced by human cells.

Thus, the chemical modification of proteins by PAG allows the development of both non-human proteins and human recombinant proteins usefull in human therapy.

It is thus not surprising that the chemical modification of proteins by PAG chains has focused much attention in the last few years and that the number of patents covering this new area is rapidly growing.

From a chemical point of view, PAG contains two types of chemical function : ether bonds and alcoholic functions. Thus, by itself, PAG does not react spontaneously with the chemical functions (amino, carboxylic acid, thiol, guanidine, alcohol) naturally present in proteins.

As a consequence, it is a common practice to "activate" the otherwise inert PAG molecules by derivatizing the hydroxyl functions present at both extremities of the PAG molecules. Such an activated PAG molecule is then capable to covalently bind to proteins. Such activated PAG molecules are also capable to realize crosslinkings of protein molecules leading to protein oligomers. When the obtention of protein oligomers is considered as a nuisance, it is still another common practice to use monosubstituated polyalkylene glycol (mPAG) as starting material.

The number of ways to achieve PAG activation is theoretically nearly unlimited and an extensive review of all the chemical means which have or could be used is outside of the scope of this summary.

Most of these PAG derivates are designed to react with primary amino function generally present in great number at the proteins surface and thus easily accessible for reactions. There exist few exceptions of PAG derivatives designed to react with other function such as those of the arginyl (guanidine function) and the cysteinyl (thiol function) residues generally present in proteins at lower levels. The chemical stability, under physicological conditions of the bonds between the PAG molecules and the protein is another characteristic met by most of these derivatives with very few exceptions.

Thus most of the known PAG derivatives, alone or in combination already meets more or less well the actual demand for the building of useful derivatized proteins, but they suffer the following drawbacks :
1. The need of a protein high polyalkylene glycol substitution level, may lead to the loss of their biological activities. When the protein has been produced by the recombinant technology, a compromise however may sometimes be found. The case of recombinant Interleukin-2 (rIL-2) may be cited as a typical example : rIL-2 has an initial very short plasmatic half-live, of about being 13 minutes initially after intravenous bolus injection followed by a slow elimination phase with a plasmatic half-live of 85 minutes. On the other hand, antibodies to rIL-2 have been found in patients undergoing clinical trials with rIL-2. High levels of modification of rIL-2 by PEG (polyethylene glycol) abolished the activity of this cytokine. A compromise was found and resulted in the evaluation of a rIL-2 derivative (PEG-IL-2) containing 2 to 3 PEG (Mr:7KDa) molecules/rIL-2 molecule with an in vitro specific activity 2- a 3 times lower than that of unmodified rIL-2. However, the in vivo activity of PEG-IL-2 was significatively higher due to its extended, between 10 and 20 times, plasmatic half-live. Moreover the immunogenicity of rIL-2 was immunosupressed by PEG-IL-2 (Katre, 1990, J. Immunology, 144 : 209-213; Mattijeen & al, 1992, Int. J. Cancer, SL : 812-817). On the other hand, non-human proteins absolutely require higher PAG substitution levels to be immunologically safe. For those non-human proteins, where modification by PAG produces a great loss of biological activity, no such compromise may be found. This drastically limits the number of proteins which may be treated according to the current PAG technology.
2. The PAG derivatives currently used to modify the protein amino and/or guanidine functions are not able to selectively react with individual side chain residues. In other words, the individual reactivities of each amino functions in a protein for example are very close to each other. The reactivity of amino and guanidine functions is very similar since most of the protein functions are located near the surface of the polypeptide chains and thus are under the influence of a similar microenvironment mainly produced by the contributing solvent molecules (water). Thus, partial modification of proteins by known PAG-derivatives results in an extremely heterogeneous mixture of different molecular species.
   Furthermore, PAG being themselves an heterogeneous polymer, this increases even more the heterogeneity of the obtained modified proteins. It has been calculated that a protein, such as horse heart myoglobin, for example, which contains about 20 reactive amino functions when, derivatized with a PEG of moderate distribution (Mr 8 to 10 kDa) may result in a mixture of more than 10³³ different molecule species.
   It is obvious that such an amount of distinct PAG-protein molecules renders the analysis extremely difficult, costly and certainly practically impossible. This challenging analytical problem, which leads to improper characterization of PAG-proteins, will be the source of additional administrative difficulties when seeking for market approval by governmental authorities such as the Food and Drug Administration in the United States per example.
3. Most, if not all, of the PAG-derivatives used to modify the protein amino functions are susceptible to hydrolyse, with as a consequence a competition reaction between hydrolysis and aminolysis. Consequently, to achieve a given level of protein modification by PAG, an excess of PAG-derivative is needed to be introduced at the start of the reaction. Excesses of up to 10 moles of PAG-derivative per mole of amino function have been reported. The separation of the products resulting from hydrolysis of the excess of activated PAG (sometimes toxic) from the PAG-protein conjugates becomes a real challenging problem of purification. This may easily jeopardize the process.
4. As of today, only irreversible protein modifications by PAG, are of interest and reversibility, when observed, is considered as a nuisance. Irreversibility here means "not reversible under physiological conditions" or in other words the PAG molecules remain covalently attached to the protein under physiological conditions and reversibility meaning the restitution of the initial protein under physiological conditions.
   Even when the chemical bond linking the PAG molecules to the protein is not stable, reversibility is not necessarily achieved. For example, the PEG-protein conjugate (the protein here is representend by P-NH₂) obtained from the reaction of the PEG-succionyl N. hydroxysuccinunide ester PEG-O-CO-(CH₂)-(CH₂)-CO-O-succinunide which formula is : PEG-O-CO-CH₂-CH₂-CO-NH-P is not stable.
   Upon hydrolysis this unstable PEG-protein conjugate leads to the starting PEG material (PEG-OH) and the succinylated protein (P-NH-CO-CH₂-CH₂-CO₂^{⊖}) which, obviously, is not the starting protein.
   To limit the research to the building up of irreversible PAG-protein adducts is a self-limitation which has precluded this PAG-technology to become more flexible. Activity in the area of PAG-modified polypeptides has increased over the last two decades as evidenced by growing numbers of research groups that have joined this field as well as by the total number of relevant publications and patents.
   The preceding work, briefly reviewed here, shows that much can still be achieved in the relatively mature field of PAG-derivative synthesis to produce novel PAG-derivatives with presently unavailable properties.

### Summary of the present invention

The present invention relates to new poly(alkylene glycol) derivatives able to selectively and reversibly attach poly(alkylene glycol) molecules to thiol functions.

For the purpose of this invention the reaction of modifying chemically a sulfur atom by attaching to it a derivatized poly(alkylene glycol) is defined as : "S-PAGYLATION".

Another object of the present invention is the discovery of new PAG-derivatives which can achieve the reaction of S-pagylation rapidly and stoechiometrically. The reaction speed is essential to such reactions since it influences the cost effectiveness on one side. On the other hand for more sensitive thiol containing molecules, such as proteins for exemple, high reaction rates will diminish drastically the risks of destruction. The stoechiometric and quantitative conversion has the double advantage of modifying all the sulfur atom present in the molecule and by doing so to supress all the physicochemical properties related to the presence of these sulfur atoms, and on the other hand it will assure a total protection of all the sulfur atoms, due to the total blockage of all of them, preventing them to undergo any further unwanted reaction such as oxydation. Reversibility will assure that the S-pagylated material will properly restore, when reconverted to the initial sulfur compound, all the initial thiol functions.

A first PAG-derivative usefull for S-pagylation of thiol derivates for the purposes of this invention are the poly(alkylene glycol) organomercuric derivates with general formula :

PAG - ( O - V - Hg^{⊕}X^{⊖} )_{n=1,2} (I)

where X is a counter - anion such as Cl^{⊖}, CH₃COO^{⊖}, OH^{⊖}, ... and V is a variable region linking the PAG to the atom of Mercury. V is a non polymeric organic group.

Examples of such poly(alkylene glycol) organomercurial derivatives include : 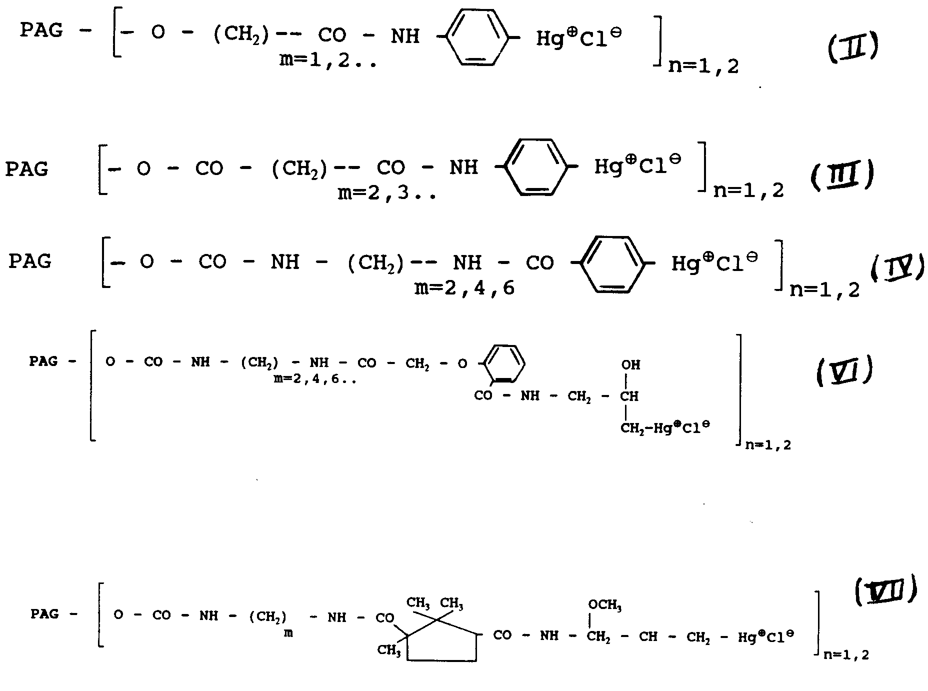
where n = 2 if poly(alkylene glycol) is used and n = 1 if monosubstituted poly(alkylene) is used as starting material.

Thiol functions are quantitatively modified with PAG-chains through mercaptides formation by adding stoechiometric amounts of PAG-organomercuric derivatives in accordance to the following reaction :

P - SH + PAG - ( O - V - Hg^{⊕} X^{⊖})_{n=1,2} → PAG - ( O - V - Hg - S - P )_{n=1,2}

Modification of thiols is carried out, at room temperature, in aqueous solutions (pH 4 to 8). Mercaptide formation is obtained quantitatively by adding stoechiometric amounts of any of these PAG-organomercurial derivatives. The reaction is completed within minutes following the addition of the PAG-organomercuric derivative.

Identical reactivities are observed when using, as starting materials, PAG characterized by different chain lengths. For that purpose Monomethoxy Poly(ethylene glycol) organomercurial derivatives of formule (I) containing PEG chains with nominal molecular weights between 750 and 20 000 Daltons were successfully tested.

Bifunctional PAG organomercurial derivatives (n = 2) were similarly obtained. They showed a quite similar pattern of reactivity towards thiol compounds.

The sulfur containing can be regenerated easily from the organomercuric pagylate thio derivative by incubating the mercaptide in an acid buffer at pH of about 3 or in an alkaline buffer of pH of about 8. The separation of the poly(alkylene glycol) moiety from the sulfur containing molecule being performed using known separation technics such as molecular sieving using per example Sephadex G - 50 supports and elution solvants such as acetic acid buffers.

PAG-organomercuric derivates of formula (II) are obtained following Scheme 1 : 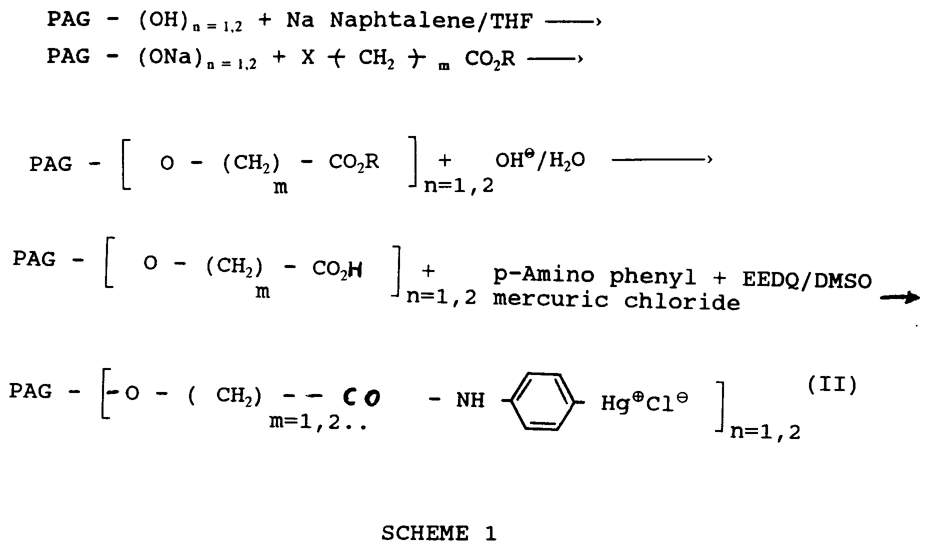
To a T.H.F. solution of sodium poly(alkylene glycolate), resulting from the reaction of poly(alkylene glycol) with sodium naphthalene, is added an halogeno acid ester. After hydrolysis a poly(alkylene glycol) acid is obtained. This acid in solution in dimethyl sulfoxide reacts at room temperature during 24 h with p-amino phenylmercuric chloride in presence of 2-Ethoxy-1-ethoxycarbonyl-1,2-dihydroquinoline (EEDQ). After precipitation with dimethyl ether the poly(alkylene glycol) organomercurial derivative is filtered and dried under reduced pressure in presence of P₂O₅. The product may further be purified by elution on a Sephadex G - 50 preequilibrated and eluted with water. Dry product can be obtained by lyophilisation. The value of m in formula (II) is function of the halogeno acid selected such as m = 1 for 2-Bromo acetic acid; m = 2 for 3-Bromo propionic acid, when monomethoxy poly(alkylene glycol) is used, n = 1 and when poly(alkylene glycol) is used as starting materials n = 2.

Poly(alkylene glycol) organomercuric derivatives with formula (III) may be obtained according to SCHEME 2. 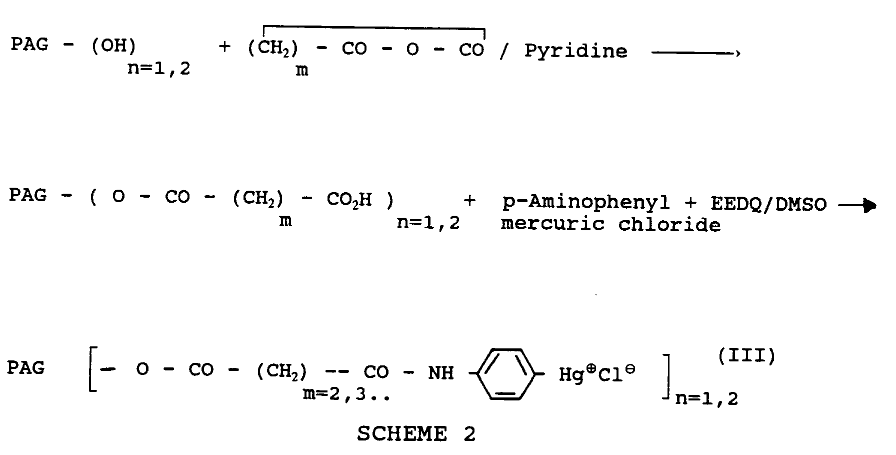
The poly(alkylene glycol) acid is obtained by reacting poly(alkylene glycol) with an organic dicarboxylic anhydride in presence of pyridine. Pyridine is removed by rotaevaporation and after precipitation of the acid in diethyl ether, the product is filtered and the condensation with paraaminophenyl mercuric chloride is performed as for the synthesis of product (II).

Saturated, unsaturated and substituted dicarboxylic acid anhydride may be used. Example of such anhydride useful for the purpose of this invention include succinic anhydride (m = 2), glutaric anhydride (m = 3), pimelic anhydride (m = 4).

PAG (polyalkylene) organomercuric derivatives of formula (IV) may be obtained according to SCHEME 3. 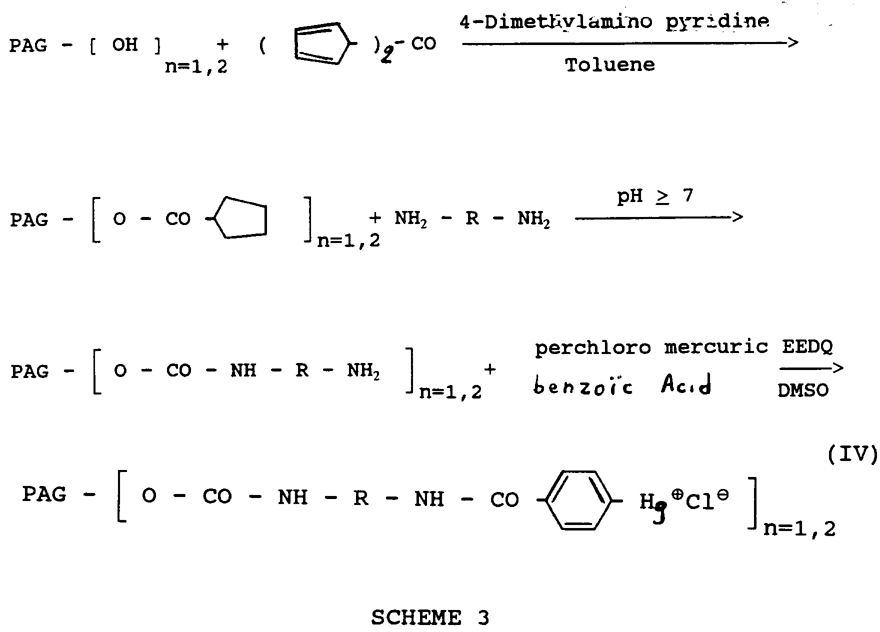
Carbonylimidazol-1-yl-poly(alkylene glycol) is quantitatively synthesized from N,N-carbonyldiimidazole and polyalkylene glycol in the presence of a stoechiometric amount of 4-dimethyl-aminopyridine used as hypernucleophilic catalyst. A diamine is then reacted with carbonyimidazol-1-yl poly(alkylene glycol) at room temperature in water buffered at pH 7 or higher. The resulting amino-PAG is purified (e.g. using dialysis to remove excess of the diamine) and conjugated to p-chloromercuribenzoic acid in the presence of E.E.O.Q. (or a carbodiimide) to obtain the PAG organomercuric derivatives of formula IV.

Diamines such as ethylene diamine (R = 2) tetramethylene diamine (R = 4) hexamethylene diamine (R = 6) or other substituated diamines may be used.

PAG (polyalkylene) organomercuric derivatives of formula in and VI may be similarly obtained starting from the amino PAG derivatives as obtained in scheme 3 and the appropriate organomercurials containing a carboxylic acid function.

Disulfides, on the other hand, are generally obtained by air oxidation of thiols according to the equation : 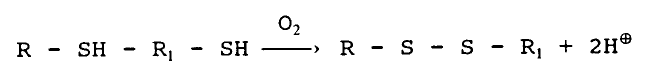
which also leads to the by-products R-S-S-R and R₁-S-S-R₁ where R and R₁ represent alkyl or aryl groups. Generally speaking air oxidation of thiols is known to be catalyzed by mixtures of thiols and disulfides as well as by di- and tri-valent metallic cations such as cupric ions. However, even in the presence of such catalytic systems, air oxidation is a slow process requiring several hours to achieve a complete reaction.

Other chemical means may be used to accelerate thiol oxidation. Such means make use of oxidants such as iodine or hydrogen peroxide. However, by doing so, the formation of the by - products R-S-S-R and R₁-S-S-R₁ can not be avoided with the consequence that the yields of obtention of the desired disulfide R-S-S-R₁ are very low.

In another aspect of the present invention, PAG disulfide derivatives able to react rapidly and stoechiometrically with thiol containing molecules (P-SH) were discovered. These derivatives react by quantitatively forming the awaited asymmetric disulfide PAG-S-S-P and permits the avoidance of the formation of the unwanted symmetric disulfides : P-S-S-P and PAG-S-S-PAG.

PAG disulfide derivatives fulfilling the requirements of the present invention have the following formula :

PAG-(O-V₁-S-SR₁)_{n=1,2} (VII)

where V₁ is a variable region linking the PAG to a sulfur atom. V₁ is a non polymeric organic group, and R₁ is selected from the group of -SO₃^{⊖}; -S-SO3^{⊖}; -SO₂CH₃; thiopyridyl; nitrosulfenyl; otherwise substituted sulfenyl; -SO-(CH₂)ₘ-CH₃ with m greater or equal to 0 (m = 0, 1, 2, ...); -SOₐ-CH₂-CH=CH₂ with a = 1, 2; -SO₂-R where R is alkyl or aryl.

Examples of such PAG-disulfide derivatives are illustrated below. 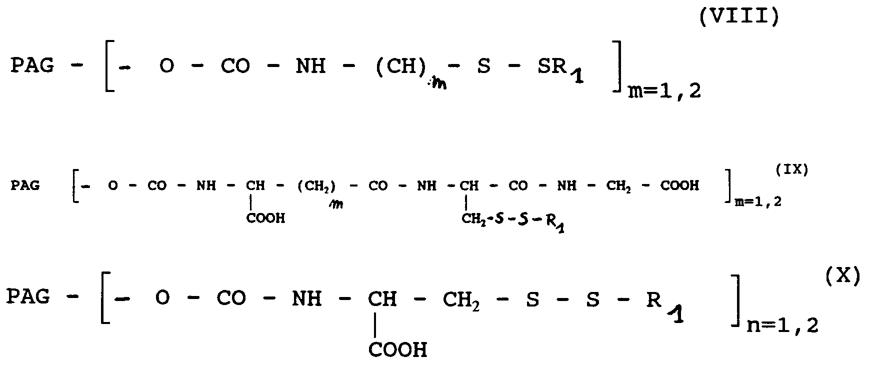
wheres R₁ has the same value as for (VII).

Modification of the thiol containing compounds through mixed disulfide bond formation take place at room temperature in aqueous buffers (pH 5 to 9). Completion of the reaction is reached within minutes after the stoechiometric addition of the PAG-disulfide derivatives (VII) of the invention.

In some cases, the reactions can be followed spectrophotometrically. As an example, 2-thiopyridone which is liberated in the course of reaction of some derivative of the invention with let's say glutathione (in the reduced form) absorbed at 343 nm in a UV region where nor the said derivate nor glutathione absorbed.

As previously observed with the organomercurial PAG-derivatives, the chain length of PAG here also does not affect the pattern of reactivity. This is also true for the bifunctional derivatives obtained when using PAG instead of monosubstituted PAG as the starting material.

In was discovered and this is another object of the present invention, that poly(alkylene glycol) derivatives containing a free thiol function reacted very easily and quantitatively with the thiol functions of the compound to be S-pagylated are activated.

Examples of such activated thiol functions are illustrated below and included derivatives such as : E representing the compound to be pagylated :

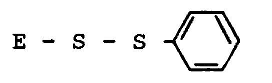

E - S - S - SO₃^{⊖}

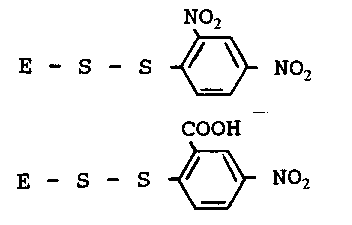

These derivatives can be readily obtained by treating E-SH with respectively : 2,2-dipyridyldisulfide, sodium tetrathionate, 2,4-dinitrophenylsulfenyl chloride and Ellman's reagent (5,5'-dithiobis-2-nitro)-benzoic acid.

Such derivatives are then capable to react stoechiometrically, rapidly, quantitatively and specifically (under mild physiological conditions) with PAG-derivatives containing a free thiol function : in accordance with the following reaction :

E - S - S - R + PAG (SH)_{1,2} - S → E - S - S - PAG + R - S - SH (XI)

Examples of poly(alkylene glycols) containing free thiols useful for the present invention are : 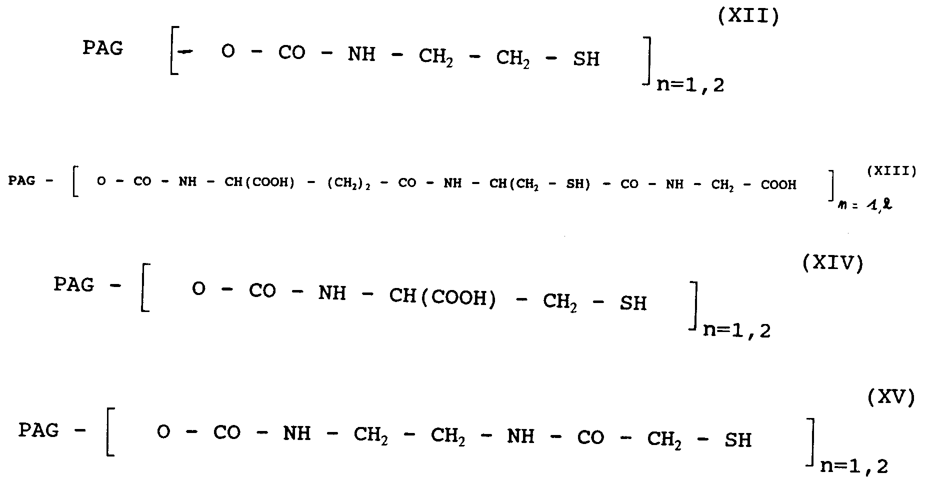
when monomethoxy poly(alkylene glycol) is used n = 1 and when poly(alkylene glycol) is used n = 2.

It should be pointed out that S pagylation of thiol containing enzymes or proteins by means of thiol containing poly(alkylene glycol) can only take place if the enzyme or protein is previously "activated".

Poly(alkylene glycols) thiol can easily be obtained by known synthetic methods such as :
1. Reaction of poly(alkylene glycol) tosylate with sodium sulfide in water.
2. Reaction of poly(alkylene glycol) tosylate with thiourea followed by alkaline hydrolysis.
3. Reaction of poly(alkylene glycol) tosylate with sodium trithiocarbonate followed by acid hydrolysis.

Commercial preparation of m PAG always contain some bifunctional PAG. When such commercial m PAG material is used to irreversibly modify peptides and proteins, some cross linkings which inevitably occurres, turn out to be a real nuisance. When reversible modification is programmed, such as in the present invention, this problem is less acute since it is essentially transitional.

The present invention also describes some among the many opportunities offered by the use of these new derivatives, in the field of the PAG technology, to the protein chemists as well as to the pharmaceutical industry.

The derivatives discovered in the present invention are useful in a large variety of fields such as protection of thiol containing molecules from oxidation or any other degradation process, isolation of thiol containing chemical compounds from complex mixtures and a synthetic intermediates. The protein chemistry field is a preferred area where to apply the teachings of the present invention.

Thiol functions in peptides and proteins naturally derived from the side chains of cysteinyl residues when present in such peptides and proteins. Generally speaking, the number of thiol functions in peptides and proteins is rather low as compared to the number of other functions present in such biological materials such as amino, guanidine, alcoholic or carboxylic acid functions.

Due to its powerful nucleophilic properties, the thiol functions of peptides and proteins, may often be selectively, specifically and quantitatively modified.

THUS, THE THIOL FUNCTION, IN PEPETIDES AND PROTEINS, ARE THE BEST CANDIDATES, TO SATISFY THE DEMAND FOR VERY PROPERLY OBTAINING LIMITED MODIFICATION OF PROTEINACEOUS MATERIALS BY PAG CHAINS.

On the other hand, thiol functions may be introduced in polypeptides allowing, when needed, a high level of modification by PAG chains to be obtained with all the above-cited advantages of S.-pagylation.

A first means for the introduction of thiol functions in proteins consists in reducing, when present, cysteinyl residues (in other words : the disulfide bonds). Reduction is readily achieved by using reducing agents such as NaBH₄, low molecular weight thiol compounds such as cysteine, 2-mercaptoethanol, dithiothreitol ..., by using sulfitolysis ... In some cases, reduction is facilitated when carried out in the presence of chaotropic agents such as guanidinium hydrochloride or urea which by denaturing the polypeptide chains renders accessible the disulfide bonds to be reduced.

Another means may consist using bifunctional reagents which contain a thiol function on one hand and a chemical function able to react with the amino, guanidine, alcoholic or carboxylic acid function of the protein on the other hand. Ideally, the thiol function present on the biofunctional reagent should be protected. An example of such reagents is provided by : 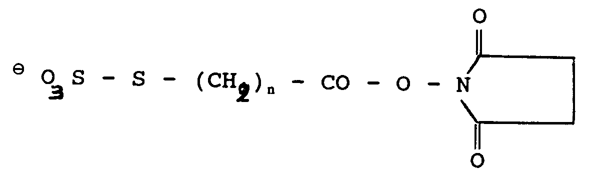
able to react with the amino functions of a protein (P-NH₂) to lead to

N-hydroxysuccinimide + P-NH-CO-(CH₂)ₙ-S-SO₃^{⊖}

from which a thiol function is readily generated.

Thiol functions may be regenerated from a great variety of derivatized thiols including mercaptides (R-Hg-S-R¹), disulfides (R-S-S-R)¹ where R and R¹ represent alkyl or aryl radicals) and some thioethers (such as S-benzyl, S-p-nitrobenzyl, S-trityl ...) among others.

Regeneration of thiols from mercaptides and disulfides thus, in addition to be quantitative, proceeds under very mild conditions (solvent : water, near to neutral pH, room temperature ...). In such mild conditions, very close to physiological media, peptides and proteins best conserved intact there biological activities.

PAG mercaptides and disulfides as described herein are products of choice, for the obtention of reversibility when this property is required.

It will be apparent to the man skilled in the art that a variety of applications may derive from the used of these PAG-derivatives. The examples cited below are only for the purpose of clarity and by no means limit the present invention.

### EXEMPLE 1

### Preparation of monomethoxy polyethylene glycol organomercuric derivatives

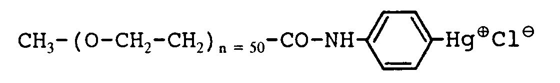

This derivative, designed here mPEG-Hg^{⊕} 2.5 has been synthesized starting from monomethoxypolyethylene glycol (Mr:2200 Da).
CH₃-(O-CH₂-CH₂-)ₙ-CO₂H was obtained as described previously by Bückmann et al (1981) Makromol. Chem. 182 : 1379-1384. This derivative (2 g; 0.91 mole) and p-aminophenylmercuric chloride (322 mg; 1 mole) were dissolved in 10 ml of dimethylsulfoxide. 2-Ethoxy-1-ethoxycarbonyl-1,2-dihydroquinoline (EEDQ, 500 mg; 2.02 mmoles) was added and the reaction mixture allowed to react at room temperature for 24 hours. The reaction mixture was then added dropwise to diethyl ether (250 ml) and the resulting suspension filtered. m PEG-Hg^{⊕} was washed with diethyl ether and dried under vacuum in the presence of P₂O₅ (yield : 1.84 g). Pure m PEG-Hg^{⊕} 2.5 was obtained by dialyzing an aqueous solution against water and dried by lyophilization.

The UV absorption spectrum of m PEG-Hg^{⊕} 2.5 resemble closely that of p-aminophenylmercuric chloride (λmax : 242 nm; ε 242 = 12800 M⁻¹.cm⁻¹; solvent : H₂O).

### EXEMPLE 2

### Preparation of monomethoxy polyethylene glycol succinyl organomercuric derivatives

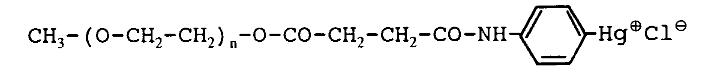

m PEG Hg^{⊕} - 5.3 was obtained in two steps starting from monomethoxy polyethylene glycol (m PEG:Mr:5kDa).m PEG (1.mmole) was first reacted with succinic anhydride (10 mmole) in dry pyridine (25 ml) for 2 hours at 60°C according to Wie et al, 1981, Int. Archs Allergy appl. Immun. 64:84-99. After having removed most of the pyridine by rotaevaporation, the reaction mixture was added to 100 ml of diethyl ether and the derivalized PEG intermediate was collected by filtration and washed with ether. The intermediate was then reacted with p-aminophenylmercuric chloride as described in example 1.

### EXEMPLE 3

When thiol containing compounds such as L-cysteine are added to solutions of m PEG-Hg^{⊕} 2.5 prepared in example 1, the absorption spectrum of the latter was modified. Batochromic and hyperchromic effects resulted from the formation of the mercaptide e.g. 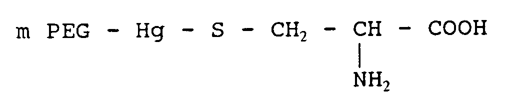
The differential spectra which resulted from mercaptide formation were characterized by a maximum at 262 nm. These spectra were used to spectrophotometrically titrate m PEG-Hg^{⊕} 2.5 solutions with L-cysteine. A ε 246 = 14770 M⁻¹.cm⁻¹ was estimated for m PEG-Hg^{⊕} 2.5.

When this value of 14770 M⁻¹.cm⁻¹ was used, m PEG-Hg^{⊕} 2.5 was found to stoechiometrically inhibit a freshly reactivated solution of papaya proteinase III, a cysteine-proteinase purified from the latex of Carica papaya L ...

### EXAMPLE 4

The condensation of the organomercurial as prepared in example 1 was then performed as previously described in example 3 and m PEG-Hg^{⊕} 2.5. m PEG-Hg-SCH₂CH₂OH was obtained after reaction, in water, with stoechiometric amounts of 2-mercaptoethanol and lyophilization.

### EXEMPLE 5

### Preparation of polyethylene glycol disulfide derivatives

CH₃-O-(CH₂-CH₂O)ₙ-CO-NH-CH₂-CH₂-S-S Py where Py is 2-pyridyl.

This derivative, designed here as m PEG-S-S-Py has been synthesized, in four steps.

### Step 1

### Synthesis of Carbonylimidazol-1-yl-methoxy polyethylene glycol (CI.m PEG)

m PEG (25 g; 5 moles) was dissolved in toluene (300 ml). Traces of water present in m PEG (0,4% as measured by the Karl-Fisher method) and in toluene were removed by the azeotropic distillation of 100 ml of solvent. To the m PEG solution, cooled to room temperature, 1,1'-carbonyldimidazole (892 mg; 5.5 moles) and 4-dimethylaminopyridine (5 moles) were added. The reaction was allowed to proceed at 25°C for 24 hours. The reaction mixture was then concentrated by rotaevaporation under vacuum and poured into 900 ml of diethyl ether. The resulting suspension was maintained under agitation for 20 hours and CI-m PEG was then collected by filtration, washed with diethyl ether and finally dried under vacuum in the presence of P₂O₅. Typical preparations were characterized by ε values at 231 nm higher than 3410 M⁻¹ cm⁻¹ which indicated CI-m PEG contents higher than 95%.

### Step 2

Cystamine dihydrochloride (2.25 g; 10 mmoles) was dissolved in water (25 ml) and the pH of the solution was adjusted to 7.0 with 5N NaOH. Solid CI-m PEG (5 g; 1 mmole) was added and the reaction allowed to proceed at 25°C for 24 hours. Excess of cystamine, NaCl and imidazole were removed by dialysis (visking tubes; cut off : 12-14 kDa) against water.

### Step 3

Dithiothreitol (1.54 g; 10 mmoles) was added to the dialysate and the pH adjusted to 8.0 with 5N NaOH. Reduction proceeded for 30 minutes at 25°C. Excess of DTT and of the by products of the reaction were then eliminated using dialysis against water. The dialysate was then concentrated by rotaevaporation up to 10 ml.

### Step 4

The concentrated dialysate was then added dropwise to a stirred solution of 2,2'-dipyridyl-disulfide (1.10 g; 5 mmoles) in dimethylsulfoxide (25 ml). Thiol-disulfide interchange proceeded at pH 5.0 for 30 minutes. Water was then eliminated by rotaevaporation and then azeotropically (with toluene). The dried reaction mixture was then added dropwise to diethylether (200 ml). m PEG-S-S-Py was collected by filtration, washed with ether and finally dried under vacuum in the presence of P₂O₅.

The spectrum of a 128.6 µm in water of m PEG-S-S-Py is characterized by a first maximum at 281 nm (ε 281 = 4900 M⁻¹ cm⁻¹) and a second one at 231 nm. Upon reaction with L-cysteine, this UV spectrum is converted into that of 2-thiopyridone with a first maximum at 272 nm and a second one at 343 nm (ε 343 = 7700 M⁻¹ cm⁻¹).

These measurements of A281 and A343 provided the basis for both the spectrophotometric determination of concentrations of m PEG-S-S-Py and the follow up of its reaction with thiol functions by measuring concentrations of liberated 2-thiopyridone.

### EXEMPLE 6

### FACILE ISOLATION OF CYSTEINYL - CONTAINING PEPTIDES TROUGH SELECTIVE FORMATION OF PAG-PEPTIDE CONJUGATES

This example describes the usefulness of the S-pagylation technique in the recovery of thiol containing peptides from a mixture of peptide. Therefore a mixture of cysteinyl containing peptides, from which the thiol functions are blocked, and a free thiol containing peptide (glutathione) was prepared. The thiol containing peptide was isolated from this mixture using the S-pagylation technique.

Reduced glutathione (γ-L-glutamyl-L-cysteinyl-glycine), the thiol containing peptide, was added to a mixture of peptides generated from reduced and S-carboxymethylated lysozyme. The resulting peptides mixture was submitted to S-pagylation using m PEG-S-S-Py (prepared as in example 5) and then fractionated on a Sephadex G 50 column. By molecular sieving, the S-PEG-glutathione conjugate, which eluted in the void volume, was readily separated from all the unpagylated peptides. Glutathione was then regenerated by reducing the disulfide bond linking PEG to the peptide.

### Preparation of the blocked protein sample

Henn egg white lysozyme (145 mg; 10 µmoles) was dissolved in 100 nM tris-acetate buffer at pH 8.5 containing 7M guanidinium hydrochloride (20 ml) and submitted to denaturation for 4 hours at room temperature. To reduce the disulfide bonds dithiothreitol (154 mg; 1 mmole) was then added. Reduction, which proceeded for 2 hours at room temperature was followed by the addition of iodoacetamide (407 mg; 2.2 mmoles) while maintaining the pH at 8.5 by the addition of 100mM tris base (30 minutes). The reaction mixture was then dialysed against water. Reduced and S-carboxymethylated lysozyme, which precipipated in the course of dialysis, was collected by centrifugation (35000 g; 30 minutes; 4°C). Reduced and S-carboxymethylated lysozyme was resuspended in 10 ml of 50 mM NaHCO₃-Na₂-CO₃ buffer at pH 9.5.TPCK-treated trypsin (3 mg) was then added. Trypsinolysis proceeded at 37°C for 4 hours while maintaining the pH constant to 9.5. The solution was then acidified to pH 5 and submitted to diafiltration (cutt off 10 kDa) The volume of the ultrafiltrate was reduced to 10 ml by rotaevaporation.

### Preparation of the peptides mixture and S-Pagylation

Reduced glutathione (10 mg; 30 µmoles) followed by m PEG-S-S-Py (155 mg; 30 µmoles) were then dissolved in the concentrated solution of peptides prepared.

### Separation of the thiol containing peptides from the peptide mixture

Fractionation was carried out at room temperature on a column (2.6 x 96 cm) of Sephadex G-50 preequilibrated and eluted with 50 mM acetic acid. Elution proceeded at a flow rate of 30 ml/h. The glutathione conjugate eluted in the void volume, as m PEG-S-S-Py also did, and thus was freed from contaminating peptides generated from lysozyme which eluted much more later. Fractions containing PEG were pooled together and lyophilised. Amino acid analysis, after performic oxidation, revealed the presence of glutamic acid, cysteic acid and glycine in the expected ratio.

### EXAMPLE 7

S-PAGYLATION AS AN ALTERNATIVE TECHNIQUE TO COVALENT CHROMATOGRAPHY FOR THE PURIFICATION OF FREE THIOL CONTAINING PROTEINS.

### ANALYTICAL METHODS

### DETERMINATION OF AMIDASE ACTIVITY

Amidase activity was measured using N-L-Benzoyl-DL-Arginine-p-nitro-anilide (BAPNA) as the substrate.
a. ASSAY METHOD N° 1 : SMITH ASSAY :
   The sample is added to "Buffer 1", aqueous sodium phosphate buffer (100 mM), pH 6.0 containing EDTA (1 mM) and Cysteine hydrochloride monohydrate (10 mM), to a final volume of 1.0 ml. The enzyme (where present in the sample) is allowed to activate for 5 minutes at 37°C before the reaction is started by the addition of 4 ml of BAPNA (1.25 mM) substrate solution preheated to 37°C.
   Substrate solution is prepared by dissolving 300 mg BAPNA in warm dimethylsulfoxide, adding the solution slowly to 450 ml Buffer 1 preheated to 37°C and the making up to 500 ml with further Buffer 1.
   Substrate solution is maintained above 30°C to prevent BAPNA precipitation.
   Incubation at 37°C is continued for 30 minutes and then the reaction is stopped by the addition of 1 ml acetic acid (4N). Released 4-nitroaniline is determined by measurement of ΔA₄₁₀.
   One unit of activity corresponded to the release of 1 picomole of 4-nitroaniline (ε = 8800 M⁻¹.cm⁻¹; B.F. Eerlanger, N. Kokowsky and W. Cohen (1961), Arch. Biochem. Biophys, 95 : 271-278) per second under these conditions. These assay conditions are used to measure amidase activity of pharmaceutical preparations of chymopapain marketed throughout the world, for example chymopapain sold under the trade name Chymodiaction by the Boots Company PLC, UK and chymopapain sold under the trade name Disken by Sinpoong in South Korea. These activity units are internationally recognized and are commonly referred to as "Smith BAPNA Assay Units".
b. ASSAY METHOD N° 2 :
   Each sample is added to aqueous sodium phosphate buffer (100 nM), pH 6.8, containing EDTA (1 mM) and either dithiothreitol (2 mM) or cysteine (4 mM) to a final volume of 0.975 ml. The enzyme (where present in the sample) is allowed to activate for 5 minutes at 40°C before the reaction is started by the addition of 25 µl of BAPNA (100 mM) in dimethylsulfoxide. Incubation at 40°C is continued for 10 minutes and then the reaction stopped by the addition of 1 ml, aqueous sodium chloracetate (100 mM)/sodium acetate (200 mN) buffer, pH 4.3.
   Released 4-nitroaniline is determined by measurement of ΔA₄₁₀. As in the Smith Assay, one unit of activity, here, corresponded to the release of one picomole of 4-nitroaniline (ε = 8800 M⁻¹.cm⁻¹) per second under these experimental conditions.
   The assay conditions correspond exactly to those described by D.J. Buttle and A.J. Barrett (1984), Biochem. J., 223 : 81-88 and were used in experiments described in Examples 21, 23, 26 and 31 in W0901356-1 filed in the name of the Boots Company PLC.
c. ASSAY METHODS N° 3 :
   The sample to be analyzed (x ml) is preincubated at 40°C for 10 minutes with (1.8-x) ml of the activation buffer which contains phosphate, citrate and borate 100 mM each in addition to 25 mM L-Cysteine and 15 mM EDTA (pH 6.8). The reaction is then started by the addition of 200 µl of 10 mM BAPNA in dimethyl-sulfoxide and stopped after 15 minutes following the addition of 500 µl 30% acetic acid.
   The release of 4-nitroaniline is determined spectrophotometrically using ε 410 = 8800 M⁻¹.cm⁻¹, x being chosen so that ΔA₄₁₀ after 15 minutes never exceeded 0.80.
   One unit of activity (n Kat) is the amount of enzyme that hydrolyse one nanomole of substrate per second under the above conditions. The specific activity is expressed as the number of units per mg of protein.
   These assay conditions correspond exactly to those described by T. Dubois, A. Jacquet, A.G. Schnek and Y. Looze (1988a) Biol. Chem. Hoppe. Seyler 369 : 733-740.

### DETERMINATION OF PROTEIN CONCENTRATION

Protein concentrations were determined spectrophotometrically using A₂₈₀, 1% = 25.0 for papain, 18.3 for chymopapain and PP III (Robinson, 1975, Biochemistry, 14 : 3965-3700) and 17.8 for PP IV.

### INFRARED SPECTROSCOPY

Spectra were recorded with a Perkin-Elmer infrared spectrometer 983 G equipped with a Perkin-Elmer microspecular reflectance accessory (ref. P.E. 221-0357). The internal reflection element was a Germanium ATR plate (50 x 20 x 2 mm, Harrick EJ 2121) with an aperture angle of 45° yielding 25 international reflections. The mean stan rate was 15.5 cm⁻¹/minute and up to 20 accumulations were performed when necessary to improve the signal to noise ratio. The spectrophotometer was continuously purged with air dried on a silica gel column (5 x 130 cm) at a flow rate of 7 l/minute. Spectra were recorded at a nominal resolution of 2 cm⁻¹ and encoded every 0.5 cm⁻¹. At the end of the scan, they are transferred from the memory of the spectrophotometer to an Olivetti M 40 itself coupled to an Olivetti M 24 computer Spectra were Fourier transformed and stored for subsequent treatments.

Papain and papaya proteinase III (PP III, formerly papaya proteinase Ω), two cysteine-proteinase from the latex of Carica papaya L. were chosen to illustrate the usefulness of S-pagylation for the purification of free thiol containing proteins.

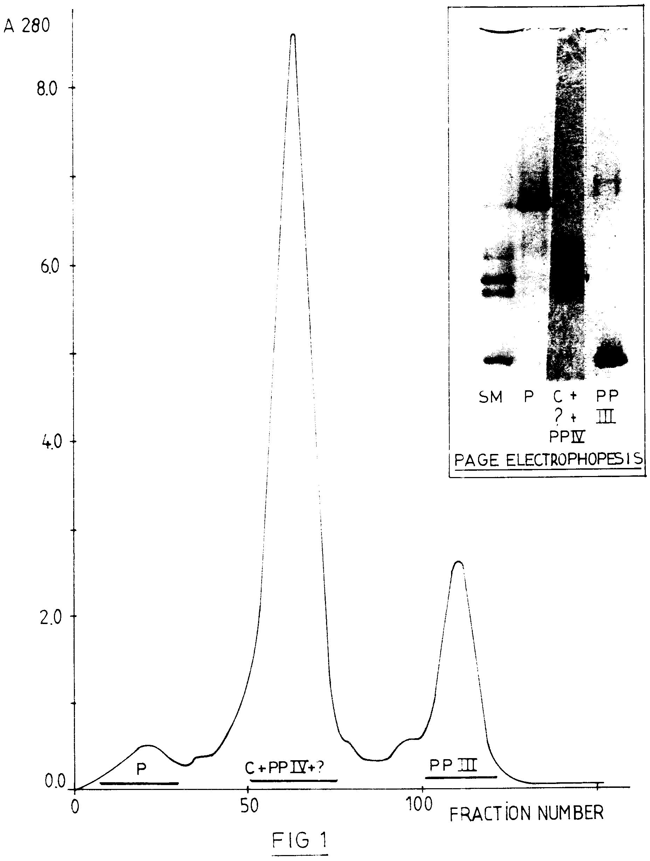

They are purified from commercially available papaya latex (Enzymase International S.A.) as follows : 2 g of spray-dried latex and 100 µl of methylmethane-thiolsulfonate were dissolved in water (25 ml). This solution was then submitted to dialysis (visking membranes with a cut off of 12-14 k Da) against deionized water (5 liters) for 6 h at room temperature before to be applied on top of a CN-Sephadex-C-50 column (25 x 150 mm) preequilibrated with 50 mM acetate buffer at pH 5.0 (the molarity of acetate buffers refers to the Na^{⊕} concentration). Elution of the bound proteins was carried out with a linear gradient from 50 to 800 mM acetate buffer (pH 5.0; total volume : 2000 ml). Elution proceeded at romm temperature at a flow rate of 60 ml/h. Fractions of 15 ml were collected and analyzed. A₂₈₀; Amidase activity; conductivity were determined in accordance with Dubois et al (1988a). Biol. Chem. Hoppe-Seymer, 369; 733-740. Figure 1 illustrates a typical elution profile from a CM-Sephadex-C-50 column of the dialyzed papaya latex in which all of the cysteine proteinases are blocked as mixed disulfides. PAGE electrophoresis results (included in Figure 1) suggest that the papain and the PP III pools were rather homogeneous in marked contrast to the second pool which contained chymopapain (C), papaya proteinase IV (PPIV) in addition to an unidentified protein material.

At this stage of purification, papain (P) and PP III preparations remained however heterogeneous at the level of the oxidation states of their thiol function. Classically, P and PP III preparations titrating one mole of SH per mole of proteinase were obtained by using covalent chromatography [e.g. Agarose-mercurial or Activated Thiol-Sepharose 4B chromatography; T. Dubois et al (1988a) Biol. Chem. Hoppe Seyler, 369:733-740]. The following results will illustrate that this technique can be substituted by a more practical procedure using the S.pagylation process of the present invention.

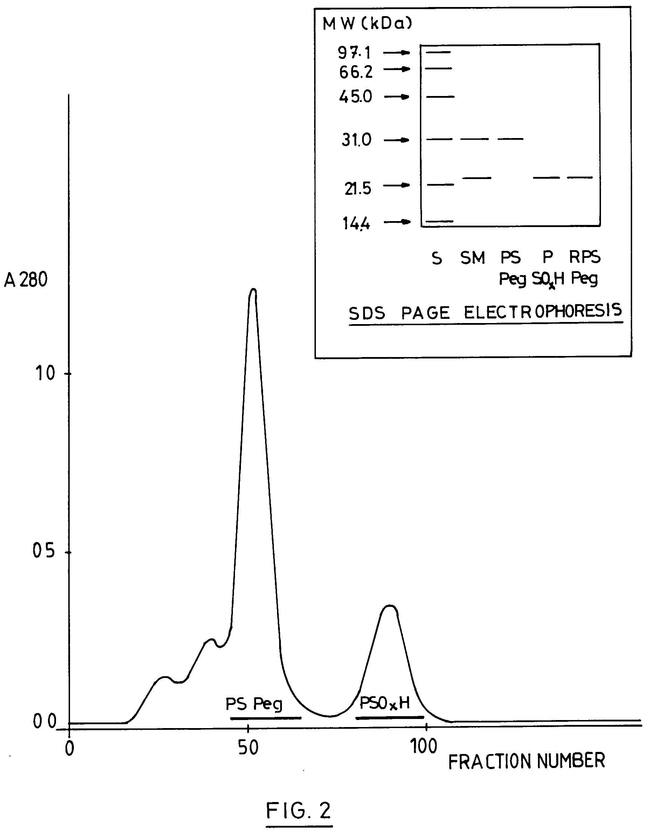

S-pegylation of papain was performed as follows : purified papain from the CM-Sephadex C-50 column (peak p of Fig. 1) was dialyzed evernight against deionized water (5 l) at room temperature and then activated using dithiothreitol (final : 5 mM) for 15 minutes. The reactivated mixture was then applied at the top of a column (10 x 100 mm) of CM-Sephadex C-50 preequilibrated with 50 mM acetate buffer at pH 5.0. After having washed the column with the preequilibrating buffer to remove the excess of reactivating agent, papain was eluted with 300 mM acetate buffer and collected in an aqueous solution containing 1.1 mole of PEG 5.S-S-Py. per mole of papain. Reaction proceeded for 1 h and the reaction mixture, after dialysis, was chromatographied on a CM.Sephadex C-50 column. Conditions of Figure 1 were used with the exception of the use of linear gradient in the range 10-300 mM. Figure 2 illustrates a typical elution profile of the mixture of irreversibly oxidized papain (PSOₓH where x = 1, 2 or 3) and S-pegylated Papain (P-S-PEG). Fractionation of PSpeg from irreversibly oxidized PSOₓH was satisfactory. Electrophoresis confirmed the molecular weight of P-S-PEG to be 5 kDa higher than the molecular weight of PSOₓH (the results of SDS-PAGE electrophoresis are included in Figure 2).

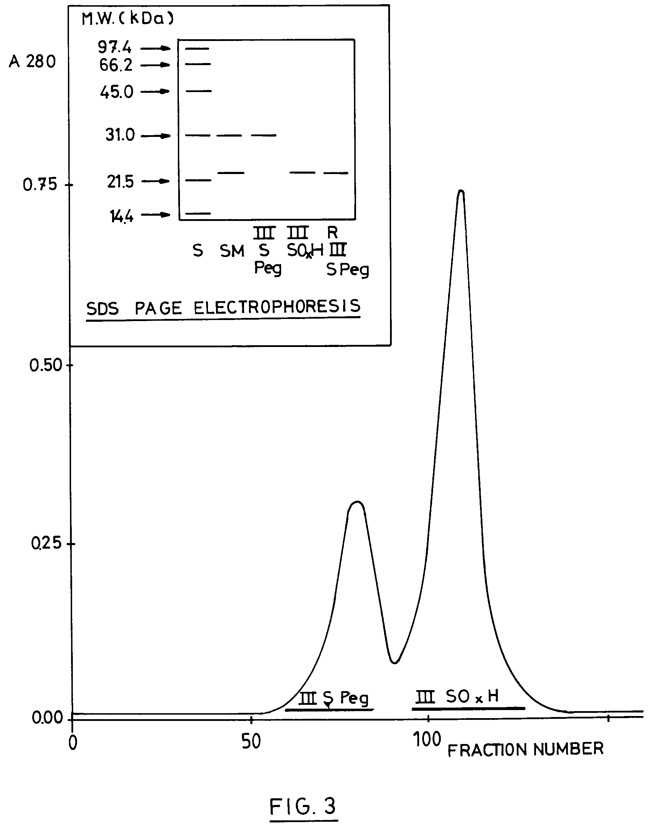

S-pegylation of papaya proteinase III was performed as for papain. The pegylated product was chromatographied on a CM-Sephadex C 50 column using a 50 mM sodium acetate buffer of pH 5.0 and a gradient 50-8000 mM. Chromatographic profiles are shown in Figure 3.

In both cases the S-pegylated protein eluted from the CM-Sephadex C-50 before the irreversibly oxidized material. This is unexpected since S-pegylation does not affect the positive charges of the protein. S-pegylation does not affect the overall structure of the proteinase. Indeed :
1. S-pegylation either of papain or PP III did not affect the contents of secondary structures of the native enzymes.
Table 1 shows the results of the Infrared spectra, the Fourier self-deconvolutions and the curve fittings of the amide I region of native and S-pegylated papain and PP III (in water at pH 5.0). The proportions of the different secondary structures were determined, as previously described by Cabiaux et al, 1989, J. Biol. Chem., 264:4928-4938, using the frequency regions for the different structures as follows : 1661-1647 cm⁻¹ : α-helix; 1689-1682 + 1637-1615 cm⁻¹ : β-sheet; 1644.5-1637.7 cm⁻¹ : random and 1682-1661 cm⁻¹ : β-turn.
From the examination of the results in Table 1, it may be concluded that the four molecular species studied here possessed identical contents of the various elements of secondary structures.

**TABLE 1**

| Original and deconvoluted Fourier Transformed Infra Red spectra of papain, P-S Peg, PP III and PP III-S Peg in the mute I' region. (Counter.ions:K^{⊕} and Cl⁻; pH 5.0) | | | | |
|---|---|---|---|---|
| | PERCENTAGE OF | | | |
| | α-Helix | β-Sheet | β-Turns | Random |
| Papain | 27.1 | 38.0 | 2601 | 8.8 |
| S-Pegylated papain | 28.2 | 3605 | 27.0 | 8.2 |
| Papaya Proteinase III | 26.4 | 39.5 | 25.9 | 8.2 |
| S-Pegylated Papaya Pectinase III | 27.9 | 36.1 | 27.7 | 8.3 |

2. The levels of amidase activity that were regenerated from S-pagylated papain and PP-III were quite comparable to those characterizing papain and PP-III preparations from affinity covalent chromatography (on columns of Agarose mercurial and activated Thiol Sepharose 4B, respectively).

**TABLE 2**

| | REGENERATED ACTIVITY* (nKat/mg) | |
|---|---|---|
| | from S-pegylation | from covalent chromatography |
| Papain | 5.41 | 5.63 |
| PP III | 1.98 | 1.87 |

| | | |
|---|---|---|
| * Amidase Assay Method N° 3. | | |

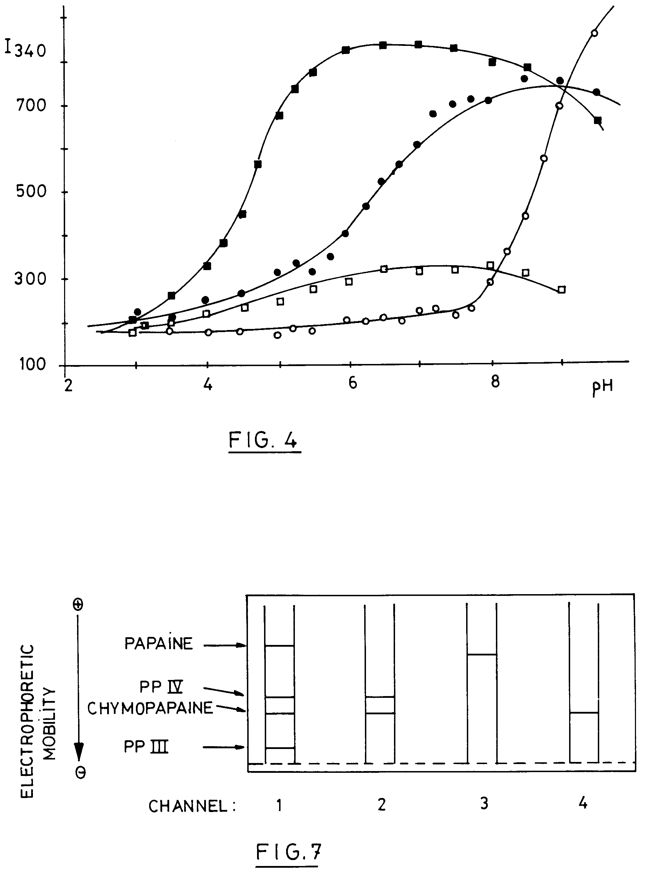

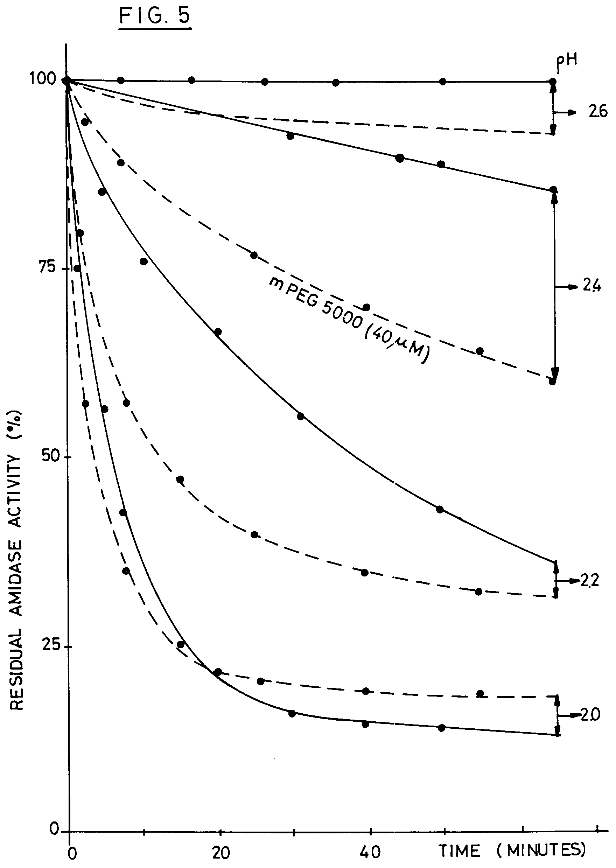

Since the chromatographic behavior on the CM-Sephadex C-50 column of the S-pegylthioproteinases, as compared to that of the unmodified proteins, remains unexplained by changes (net charge values-structural transition) of the polypeptide chain itself, we looked more closely at the level of the PEG chain as well as of its possible interaction with the protein moiety. Experimental evidence indicates that such an interaction may occur. Indeed :
1. S-pegylation of papain and PP III affected the susceptibility of these proteinases to acid denaturation. Susceptibility to acid denaturation is a property shared in common by most cysteine-proteinases belonging to the papain superfamily with the noteworthy exception of chymopapain. Acid denaturation is accompanied by an irreversible conformational transition which leads to a completely inactive and disorganized 3-D-structure.
   Experimentally, concentrated unbuffered aqueous solutions (pH 5) of the native or S-pegylthioproteinases were diluted in an acid buffer of known pH (final proteinase concentration : 40 µM) and incubated at 25°C in that acidic buffer. At various time intervals, aliquots were removed and assayed for residual amidase activity.
   As the native enzymes, the S-methylthio derivatives of papain and PP III were chosen in order to avoid a loss of activity due to oxidation or the otherwise free thiol function. By doing so the measured loss of activity was only that associated to acid denaturation.
   In some experiments, free monomethoxypoly ethylene glycol 5000 (40 µm) was added to the S-methylthio derivatives. The time course dependence of the acid denaturation of the S-methylthio and S-pegylthio derivatives of PP-III at various pH values are shown in Figure 4.
   Examination of Figure 4 reveals that :
   (i) Free methoxy PEG 5000 afforded no protection to S-methylthio PP III towards acid denaturation whatever the pH value of the buffer used.
   (ii) S-methylthio PP III solutions are stable up to pH 3.0 while S-pegylthio PP III solutions remained stable at pH values as low as pH 2.6.
   (iii) Below these critical pH values, acid denaturation, for both species, proceeded at a rate which increased with increasing proton concentrations.
   (iv) A difference of stability between the S-pegylthio PP III and the S-methylthio PP III could be observed up to pH 2.2 but not below.

   Similar results were obtained in the case of papain. It is clear that the pegylated proteins have an increased stability in Acidic Medium. The detailed mechanism of acid denaturation of cysteine proteinases remains unknown.
2. Fluorescence of papain, essentially, results from fluorescence of tryptophyl residues N° 69 and 177 in the papain chain. The fluorescence of TRP 177, which accounts for about 50% of the total fluorescence of papain, depends upon the state of ionization of the imidazole ring of histidine 159, in the active site of the enzyme, being totally quenched when His 159 ring is fully protonated.
   An apparent pKa of the imidazole ring of His 159 of about 8.5 has been measured for papain species titrating one mole of free SH per mole of protein. This value drops to around pKa 4.0 when the free SH is blocked, example of such blocked thiol is the S-methylthio derivative.
   The abnormal high pKa of 8.5 observed for the enzyme titrating one mole of SH per mole of protein is generally recognized to result from the existence of an interactive thiolate-imidazolium system in the active site of papain. On the other hand, the abnormal low pKa value of 4.0 observed when the interacting thiolate-imidazolium system is precluded, is explained by the hydrophobic environment of the imidazole ring of histidine 159. The indole ring of TRP 177 contributes to create this hydrophobic environment of the His 159 side chain.
   It has been shown that quite a similar situation can describe the case of PP III. This prompted us to measure the pH dependence of the fluorescence of the S-pegylthio derivative of PP III and to compare it with those observed for the native enzyme (titrating one mole of free thiol per mole of enzyme), the S. methylthio PP III and the irreversibly oxidized PP III.
   The S-methylthio derivative of PP III was prepared by reacting stoichiometric amounts of methylmethanethiolsulfonate and PP III prepared according to Dubois et al, 1988a loc.cit. PP III titrating one mole of SH per mole of protein was generated in the fluorescence cell from the S-Methylthio derivative by adding cysteine (5 mM) five minutes before recording the emission spectrum. The second chromatographic pooled material from Figure 3 provided irreversibly oxidized PP III sample.
   The pH dependence of the fluorescence of irreversibly oxidized PP III reported here for the first time show the pKa of His 159 of this derivative to be shifted from 4.5 to the quite normal value of 6.75 for an imidazole ring. The oxidized thiol function - SOₓH where x = 1, 2 or 3 is acidic and polar by nature as compared to -S-S-CH3. One may thus expect that the presence per se of such a function in the proximity of the imidazole ring of His 159 is capable of shifting the abnormally low pKa value of 4.5 towards a normal pKa of 6.75.
   The quantum yield of the fluorescence emission spectra of the S-pegylthio derivative of PP III was observed to be, whatever the pH, comparable to those of the quenched PP III species. On the other hand, at concentrations at least up to 50 µM, free methoxypoly ethylene glycol 5000, did not modify the fluorescence emission of the same PP III species (1 µM).
   It may thus be concluded that the covalently attached PEG chain quenches TRP 177 of the S-pegylthio derivative of PP III and that the fluorescence intensity of the quenched TRP could no more be affected by the ionization state of the imidazole ring of His 159.
   It is important to note that the quenching of TRY 177 by the covalently attached PEG chain was complete and not partial, strongly suggesting that the folding of this PEG chain was not random but rather took a more precise orientation.
3. In the course of our experiments, it was discovered that s-pegylthiopapain remained quite soluble in an aqueous solution containing 500 mM KCl.
   This behavior is quite remarkable since papain, S-methylthiopapain or other papain derivatives precipitate in such high ionic strength solutions.
   Thus, the covalent modification of papain with a 5 kDa PEG chain, clearly, precluded the modified papain molecules to aggregate.
   The mechanism responsible for this unexpected behavior remains to be elucidated, but this property will be very useful to maintain proteins solubilized when they need to be introduced in high ionic strengths mediums.
4. The circular dichroism spectra in the near UV region of the S-methylthio and the S-pegylthio derivatives of papain and PP III were compared. Circular dichroism spectra in the near UV region of the S-methylthio and the S-pegylthio derivatives of papain and PP III were recorded at pH 5.0 and 20°C with the use of a cary 61 spectropolarimeter. Quartz cells with an optical path length of 2 cm were used and contained the enzyme solutions at the concentrations of 0.4 mg/ml. For both proteinase, the modification by the PEG chain (5 kDa) was found to enhance the molecular ellipticities and thus the asymmetric character of the environment of the aromatic side chains.
   The effect was however more pronounced in the case of PP III while remaining questionable in the case of papain at least for the tyrosyl and the tryptophyl side chains (positive CD bands at 278.5 and 288 nm and negative CD band at 300 nm) and in the case of PP III for some tryptophyl side chains (negative CD band at 300 nm).
   A detailed interpretation of the CD results however is complicated by the fact that papain and PP III contain a lot of aromatic residues, respectively 28 and 19.
   Most of these aromatic residues being buried in the hydrophobic core of these proteinases the number of aromatic side chains at the surface of the proteins and susceptible to influenced by the neighborhood of the PEG chain could therefore be expected to be small. Only limited enhancements of CD bands could indeed be measured.
   These experiments show that papain and PP III 3-Dimensional structure were poorly affected by PEGylation. On the other hand, PEG exhibits the interesting property of being highly compatible with water while exhibiting strong incompatibility with a wide variety of other water-soluble substances. Incompatibility means that an unfavorable free energy change occurs when a second species interacts with a solvated PEG molecule, resulting in a statistical tendency for the second species to be excluded from the region within or near the PEG chain.
   Such excluded volume effects are manifested in a variety of ways, including phase separation in mixtures with a second water-soluble polymer or salt, enhanced exclusion of PEG from chromatographic gel beads relative to other polymers of similar molecular weight, protein precipitation and reduced binding of external proteins to surfaces or molecules derivatized with PEG.
   Such excluded volume effects were also observed here, at least, in three occasions :
   1. S-pegylthiopapain, in marked contrast to papain, remains soluble in high ionic strength saline solutions.
   2. S-pegylthio derivatives of papain and PP III offer a better resistance of acid denaturation.
   3. S-pegylthio derivatives of papain and PP III bind less strongly to CM-Sephadex C-50 supports.

   Facile methods of separation of S-pegylthio-proteinases from the irreversibly oxidized species included molecular sieving (e.g. on Sephadex G-75), hydrophobic chromatography (e.g. on Octylsepharose 4B), affinity chromatography (e.g. by using chicken cystatin as the immobilized ligand to selectively retain the irreversibly oxidized forms of the proteinases).
   Thus free thiol containing proteins can be easily purified from mixtures of even very similar (isoelectric point, molecular weight) contaminating proteins by using quite conventional procedures instead of covalent chromatography. The use of conventional procedures was made possible after the building up of a PEG-thiol containing protein conjugate linked by a disulfide bond. In particular, the irreversibly forms of cysteine-proteinases could be easily eliminated from proteinases preparations by using the PEG technology in conjunction with conventional purification procedures.
   As compared to the PEG-technology described here, covalent chromatography suffers from some drawbacks :
   1. The organomercurial support (affigel 501) presents a potential risk of toxicity due to a possible leaching of the toxic ligand from the gel matrix.
   2. Due most probably to steric constraint, not all thiol containing proteins binds to such covalent supports. PP III for example do not bind to Organomercurial support.
   3. The half-life of some supports such as Activated Thiopropyl Sepharose 6B and related gels in short due to a time course dependent irreversible oxidation of the thiol function.
   4. The free thiol containing protein is co-eluted, from covalent chromatography columns, with low molecular weight compounds consisting of a mixture of thiol and disulfide. The separation of the protein from that mixture requires additional purification steps during which while separating the protein from that mixture, some protein oxidation may occur. Generally speaking, proteinases which have been purified by this technique titrated 0.9 mole of SH/mole of enzyme instead of 1.0.
   5. The S-pegylation technic contrary to all know technics opens the way to an industrial economically protein purification process.
      Thus the PEG-technology described here, associated to any conventional purification procedure, advantageously, might replace covalent chromatography.
      This example illustrates the separation of papain and PP III from their irreversible oxydized forms. Good separations were obtained in both cases between molecular species which differs only by the oxidation state of one thiol function (SH versus SOₓH). This is really surprising if one take in account the extended large amounts or ionizable functions shored in common by both species. This demonstrates the opportunity offered by the S-pagylation of thiol containing protein for the purification of proteins even when their properties are very closely related (similar isoelectric points, molecular weight, hydrophobicity).

### EXAMPLE 8

### FACILE SEPARATION OF CHYMOPAPAIN FROM PAPAYA PROTEINASE IV THROUGH SELECTIVE S-PEGYLATION OF CHYMOPAPAIN

Highly Purified preparations of chymopapain are needed for the treatment of prolapsed or herniated discs or sciatica in a process known as "chemonucleolysis", Smith L. (1964), J. Am. Med. Assoc., 187:137-140.

Purification of chymopapain, one major constituent of the cysteine-proteinases pool from papaya latex, was first attempted by Janssen and Balls (1941), J. Biol. Chem., 137:405-417, who used acid precipitation and salting-out procedure to prepare the enzyme.

More recently, purification methods, based on ion-exchange chromatography have been employed. Such methods were described in several patents among which :
EP65395 assigned to Smith Lab. Inc.

Separation of proteolytic enzymes from carica papaya latex using Sephadex SP with a Tris-hydrochloric acid or phosphate buffer to give papain, chymopapain, lysozyme and proteinase X.

FR1592192 assigned to Baxter Labs Inc.

Chymopapain purification
FR2236702 assigned to Coop. Pharma Francaise.

Chymopapain from Carica papaya juice with anti-inflammatory activity for local and systemic use, e.g. in treating hematomas.

WO8504417 assigned to Simmons, J.W.

Chymopapain protease purification using cation-exchange resin to selectively absorb protease.

EP65395 assigned to Smith Lab. Inc.

Stable compositions containing purified chymopapain useful for administration to damaged intervertebral discs with reduced toxicity risks.

Figure 1 illustrates a typical elution profile, obtained by the applicant, of the dialyzed papaya cysteine-proteinases mixture from a CM-Sephadex C-50.

The PAGE electrophoresis (included in Figure 1) of the "chymopapain" shows clearly that at least three distinct constituents were present in that fraction. (Chymopapain + PPIV + unknown).

N-terminal amino acid analysis, showed also heterogeneity of that fraction. From this, one may conclude that cation-exchange chromatography is unable, by itself, to produce pure homogeneous chymopapain. Affinity chromatography procedures (Dubois et al, Bio. Chem. Hoppe-Seyler (1988a) 369:733-740) are able to produce a chymopapain preparation suitable for sequencing. The affinity chromatography technique used for that purpose is not suitable for application on a commercial scale, but allowed to determine the complete amino acid sequence of chymopapain without any indication of microheterogeneity [Jacquet et al (1989) Biol. Chem. Hoppe-Seyler 370:425-434]. A fourth cysteine-proteinase namely papaya proteinase IV was discovered by Buttle et al, Biochem. J. (1989), 261:469-470. It is present in papaya latex in equal amounts as chymopapain and co-elutes with chymopapain from cation-exchange resins.

The need for pure enzyme preparation is particularly important in the clinical applications of chymopapain, for example chemonucleolysis, to avoid the appearance of dangerous allergic reactions. Such adverse reactions may arise in as many as 3% of the treated patients.

Powdered papaya extract is widely used in the food industry and it is thought that many of the allergic reactions to chemonucleolysis are due to presensitization with such preparations.

In any case, it is well known that the injection of foreign protein antigens into mammals always carries with it a risk of anaphylactic shock. As a consequence it will be in accordance with sound medical practices to use the purest and most active form of any such protein which is available to obtain optimal benefits of the procedure while minimizing the risk of allergic reactions.

In experiments described by Barrett A.J., Buttle D.J. and Rich D.H., (1990) in Patent application W09013561, 26 individual serum samples were found to contain naturally acquired IgE antibodies against a commercially available chymopapain preparation Chymodiactin®, produced in accordance with the process described in GB2098997. It was too demonstrated that these sera contain IgE antibodies to the four known cysteine proteinases found in papaya latex mainly papain, chymopapain and papaya proteinases III and IV (PP III and PP IV). However, averaged IgE levels for chymopapain, PP III and PP IV have known that PP III and PP IV together account for nearly 75% of the IgE detected.

These results clearly indicate that PP III and PP IV have an important immunogenic character and may represent a major proportion of the epitopes contained in "chymopapain" preparations based on ion-exchange chromatography and create a potentially high antigenic hazard when injected in humans.

In view of the foregoing status of the art, there is an urgent need for methods to achieve purification of chymopapain up to homogeneity. A.J. Barrett, D.J. Buttle and D.H. Rich (1990) described in Patent application WO90113561, a process which purified chymopapain up to homogeneity. Their process was based on an active site directed affinity chromatography.

The following results presented here illustrate that other techniques associated to the selective S-pagylation of chymopapain have substantial advantages to their prior art procedure.

This is a further aspect of the present invention.

S-PEGylation of chymopapain was carried out, starting from the material under peak II of Figure 1; obtained by ion-exchange chromatography of the dialyzed latex, and containing chymopapain, PP IV and an unknown product. By properly pooling the chromatographic fractions of this pool, it was possible to eliminate the unknown third constituent, otherwise present, as revealed by comparing the electrophoresis profiles shown in Figures 1 (channel 3) and 7 (channel 2).

S-PEGylation of the mixture of chymopapain and PP IV was then carried out essentially as previously described in example 7 for papain and PP III.

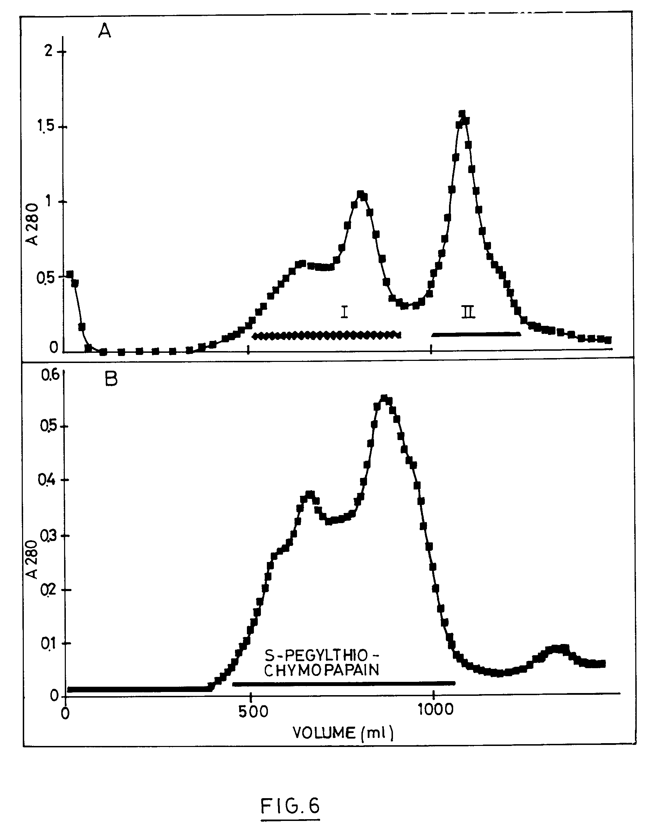

Fractionation of the reaction mixture was also obtained by chromatography on a column of CM-Sephadex C-50 using the same conditions as in example 7. A typical elution profile on CM-Sephadex C-50 is shown in Fig. 6A. The various chromatographic fractions were mixed to obtain two pools I and II as indicated by the solid bars in Figure 6.

The protein material constituting pool I was inactive against BAPNA when cysteine was omitted and active when cysteine was present.

This material also was characterized by a quite abnormal electrophoretic pattern (Figure 7 channel 3) due to the presence of covalently linked PEG. After removal of the PEG moiety (by treating with 5 mM cysteine) and its remplacement by a methylthio group, this abnormal electrophoretic behavior disappeared and revealed that peak I was constituted by chymopapain species only (Figure 7 channel 4). This clearly confirms that S-PEGylation of a mixture of chymopapain and PP IV, as expected, lead to the selective S-PEGylation of chymopapain.

The material under peak II, on the other hand, was totally inactive against BAPNA whether or not cysteine was present (5 mM) in the activation buffer. Furthermore this protein material contained chymopapain and PP IV as shown by PAGE electrophoresis which indicated on electrophoretic profile very similar to that of channel 2 of Figure 7.

PP IV is known to be inactive against BAPNA as well as against many other synthetic amide substrates (Buttel et al, 1990, loc.cit.). Thus, chymopapain which eluted under peak II was constituted of irreversibly oxidized species and indicating that S-PEGylation, as carried out here, was complete.

The S-PEGylthiochymopapain under peak I of Figure 6A was re-chromatographied on CM-Sephadex C-50 using the same experimental conditions of Figure 6A with the exception that the linear gradient applied here was from 100 to 500 mM of Sodium Acetate buffer.

The elution profile (Figure 6B) obtained in the course of this re-chromatography shows that only a very small fraction of inactive material could be further eliminated.

Microheterogeneity of the S-PEGylthiochymopapain preparation was clearly indicated by the bimodal shape of its elution profile. Such a microheterogeneity may be explained by the fact that chymopapain contains two free thiol functions located at position 25 and 117 in the polypeptide chain. Furthermore, the presence of bi-functional PEG in the commercial m PEG used to prepare PEG-S-S-Py creates an additional microheterogeneity due to possible cross-linking of chymopapain molecules.

However, upon removal of the PEG moiety and its replacement by the methylthio group, the apparent chromatographic microheterogeneity disappeared and a perfect gaussian profile was obtained from the CM-Sephadex C-50.

In this example it is demonstrated that he S-PAGylation technique offers the opportunity to purify chymopapain to homogeneity from a mixture which contains another cysteine-proteinase, namely PP IV, which shares very closely properties in common with chymopapain. This opportunity to purify chymopapain was allowed due to the possible selective S-PAGylation of chymopapain.

The separation of chymopapain from PP IV as illustrated was dictated by the technical difficulty encountered with the prior art, by the need of a very purified enzyme for medical use and by the economic importance of this enzyme.

The teaching of the present invention can be applied economically to the industrial purification of sulfur containing products and preferentially to sulfur containing biological materials.

### Figure 1

Ion-exchange chromatography of the dialyzed papaya latex on a column of CM Sephadex 50.

Column 25 x 150 mm; fractions : 15 ml; flow rate : 60 ml/h, buffer : 50 mN Sodium Acetate, pH 5.0; gradient : 50-800 mN Na^{⊕}, pH 5.0; total volume : 2 l.

P : papain; C : chymopapain; PP III and IV : papaya proteinase III and IV; ? unidentified protein material; SM starting material.

### Figure 2

Ion-exchange chromatography of the mixture of S-pegylated papain and irreversibly oxidized papain (PS PEG and PSOₓH, respectively).

Column : 25 x 150 mm; fractions : 15 ml; flow rate : 60 ml/h, buffer : 10 mM sodium acetate, pH 5.0; gradient : 10-300 mN Na^{⊕}, pH 5.0; total volume : 2000 ml.

RPS PEG reduced (2-mecaptoethanol 5 mM) PS PEG; S.M : starting material to be chromatographied. S : electrophoresis standards (see Figure 1).

### Figure 3

Ion-exchange chromatography of the mixture of S-pegylated papaya proteinase III and irreversibly oxidized papaya proteinase III (III S PEG and III SOₓH, respectively).

Column 2.5 x 15 cm, fractions : 15 ml, flow rate : 60 ml/h, buffer : 5 mM sodium acetate, pH 5.0; gradient : 50-800 mN Na^{⊕}, pH 5.0; total volume : 2000 ml.

R III S PEG : reduced (2-mecaptoethanol 5 mN) III S PEG; SM starting material to be chromatographied; S standards : rabbit muscle phosphorylase b (97.4 kDa) + bovine serum albumin (66.2 kDa) + hen egg white ovalbumin (45 kDa) + bovine carbonic anhydrase (31 kDa) + soybean trypsin inhibitor (21.5 kDa) + hen egg white lysozyme (14.4 kDa).

### Figure 4

pH dependence of the fluorescence intensity at 340 nm of PP III derivatives (1 µm) : o, PP III titrating one mole of SH per mole of enzyme, generated in situ in the fluorescence cell from the S-methylthio-derivative by adding cysteine (5 mm) five minutes before recording the emission spectrum; ● irreversibly oxidized PP III (second chromatographic pool from Figure 3; ■, S-methylthio PP III and □, S-Pegylthio-PP III (first chromatographic pool from Figure 3).

Concentrated (40 µM) unbuffered aqueous solutions of the PP III derivatives were diluted in the appropriate buffered solutions of known pH. Buffers contained phosphate, citrate and borate (final concentrations : 100 nM each) adjusted with NaOH to the appropriate pH. Emission spectra (λ excitation : 285 nm) were recorded in thermostated cells (25°C).

### Figure 5

Time course dependence of acid denaturation of S-methylthio PP III (---) and S-pegyl (5000) thio PP III (―) at various pH and at 25°C.

Concentrated unbuffered solutions (pH 5) of the S-methylthio or of the S-pegylthio PP III were diluted in an acidic buffer of known pH (final proteinase concentration : 40 µM) and incubated at 25°C. At various time intervals, aliquot were removed and assayed for residual amidase activity using the assay method N° 3.

### Figure 6

Purification of S-PEGylthiochymopapain of CM-Sephadex C-50 (Experimental conditions as for Figure 1 with the exception for Figure 6B where the gradient varied from 100 to 500 mM).
A. Chromatography of the reaction mixture resulting from S-PEGylation of a mixture of chymopapain and PP IV.
B. Re-chromatography of peak I from Figure 6A.

### Figure 7

PAGE electrophoresis of S-PEGylthiochymopapain at various stages of its purification procedure.
- Channel 1: : whole latex proteinases
- Channel 2: : protein material of pool II of Figure 1
- Channel 3: : protein material of pool I of Figure 6A
- Channel 4: : protein material as in channel 3 after conversion to the S-methylthioderivative.

## Claims

1. Poly(alkylene glycol) organomercuric derivative with general formula :
PAG [ O - V - Hg^{⊖}x^{⊕} ]ₙ
wherein PAG represent a poly(alkylene glycol), x is a counter ion such as chloride, acetate, hydroxyl and V is variable region consisting of a non polymeric organic group linking the poly(alkylene glycol) to an atom of mercuric and n = 1 or 2.

2. Poly(alkylene glycol) disulfides derivatives with general formula :
PAG [ O - V₁ - S - SR₁ ]ₙ
wherein PAG represent a poly(alkylene glycol), V₁ is a variable region consisting of non polymeric organic group linking the poly(alkylene glycol) to a sulfur atom and SR₁ is selected from the group of -SO₃^{⊕}; -S-SO₃^{⊕}; -SO₂CH₃; thiopyridyl; nitrosulfenyl; otherwise substituted sulfenyl; -SO-(CH₂)ₘ-CH₃ with m = 0, 1, ...; -SOₘ-CH₂-CH=CH₂ with m=1, 2; -SO₂-R where R is alkyl or aryl and n = 1 or 2.

3. A process consisting of selectively and reversibly attaching poly(alkylene glycol) moieties to thiol containing molecules using derivatives of claim 1 and 2.

4. A process for the extraction, separation and/or purification of thiol containing molecules consisting of :
a. attaching the PAG derivative of this molecule;
b. purifying the PAG derivative;
c. regenerating the purified thiol containing molecule.

5. A process as described in claim 4 for the extraction, separation and/or purification of sulfur containing peptides and/or proteins as well as other molecules possessing pharmacological properties.

6. A process as described in claim 3 useful to confer to thiol containing compounds novel and interesting physico-chemical properties which modulate their physiological, pharmacological and immunological status.
